(19) Europäisches Patentamt
European Patent Office
Office européen des brevets

(11) Publication number: 0 353 381
A2

(12) **EUROPEAN PATENT APPLICATION**

(21) Application number: 89104428.1

(22) Date of filing: 13.03.89

(51) Int. Cl.4: C07D 498/14 , C12P 17/18 , C12N 1/20 , A61K 31/00 , //(C12P17/18,C12R1:29, C12N1:20,C12R1:29), (C07D498/14,311:00,265:00, 221:00)

A request for correction of the structured formula on p.1 of the description has been filed pursuant to Rule 88 EPC. A decision on the request will be taken during the proceedings before the Examining Division (Guidelines for Examination in the EPO, A-V, 2.2).

(30) Priority: 04.08.88 US 179357

(43) Date of publication of application: 07.02.90 Bulletin 90/06

(84) Designated Contracting States: AT BE CH DE ES FR GB GR IT LI NL SE

(71) Applicant: AMERICAN CYANAMID COMPANY
1937 West Main Street P.O. Box 60
Stamford Connecticut 06904-0060(US)

(72) Inventor: Carter, Guy Thomas
8 Prairie Avenue
Suffern State of New York 10901(US)
Inventor: Goodman, Joseph Jacob
134 Grotke Road
Spring Valley State of New York 10977(US)
Inventor: Labeda, David Paul
2320 W. Pintura Court
Peoria State of Illenois 61614(US)

(74) Representative: Wächtershäuser, Günter, Dr.
Tal 29
D-8000 München 2(DE)

# (54) Antibiotic LL-E19085 Alpha.

(57) This disclosure describes a novel antibacterial agent designated LL-E10985a, its production by aerobic fermentation of a new subspecies of Micromonospora citrea NRRL 18351 mutants thereof, and the isolation and purification thereof.

EP 0 353 381 A2

## ANTIBIOTIC LL-E19085α

## SUMMARY OF THE INVENTION

This invention relates to a new antibacterial agent designated LL-E19085α, to its production by fermentation, to methods for its recovery and concentration from crude solutions and to processes for its purification. The present invention includes within its scope the antibacterial agent in dilute form, as a crude concentrate and in pure form. The effect of this new agent on specific microorganisms, together with its chemical and physical properties, differentiate it from previously described antibacterial agents. While not yet fully elucidated, the structure of antibiotic LL-E19085α is proposed as follows:

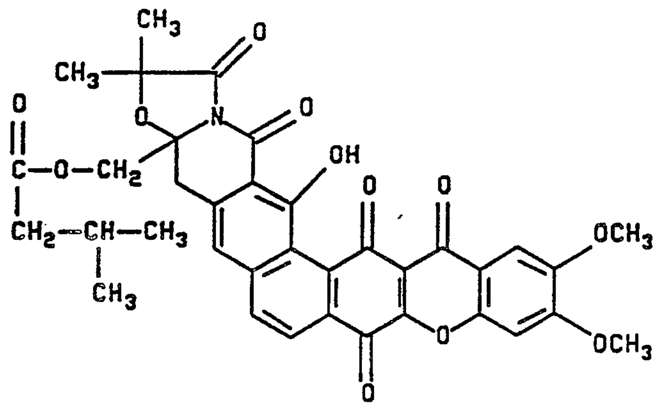

The physiochemical characteristics of antibiotic LL-E19085α are as follows:

a) Molecular formula: $C_{36}H_{31}NO_{12}$;

b) Molecular weight: 669 FABMS $(M+3H)^{+}$ = M/Z 672.21176;

c) Elemental Analysis: C, 64.55; H, 4.46; N, 1.92;

d) Specific rotation $[\alpha]_D^{26}$ = -52° (C, 1.11% - dichloromethane);

e) Ultraviolet absorption spectra: λmax nm ε 0.1N HCl = 224nm (27,700), 255nm (21,900), 328nm (16,400), 420nm (4,230)

λmax nm ε 0.1N NaOH = 217nm (76,100), 340nm (15,100), 399nm (12,900)

λmax nm ε $CH_3OH$ = 222nm (38,200), 240nm (30,100), 255nm (30,100), 321nm (24,500), 384nm (8,050)

f) Infrared absorption spectrum: (KBr disk), max ($cm^{-1}$) 1800, 1742, 1690, 1621, 1422, 1273;

g) Proton magnetic resonance spectrum: ($CDCl_3$) shows significant peaks as follows:

| δ | #H | M | J(Hz) |
|---|---|---|---|
| 13.47 | 1 | s | - |
| 8.20 | 1 | d | 8.5 |
| 7.92 | 1 | d | 8.5 |
| 7.58 | 1 | s | - |
| 7.19 | 1 | s | - |
| 7.09 | 1 | s | - |
| 4.78 | 1 | d | 11.6 |
| 4.45 | 1 | d | 11.6 |
| 4.02 | 3 | s | - |
| 3.99 | 3 | s | - |
| 3.48 | 1 | d | 12.8 |
| 3.35 | 1 | d | 12.8 |
| 2.21 | 2 | d | 6.8 |
| 2.10 | 1 | m | - |
| 1.83 | 3 | s | - |
| 1.80 | 3 | s | - |
| 0.955 | 6 | d | 6.48 |

h) Carbon-13 nuclear magnetic resonance spectrum: as shown in Figure IV ($CDCl_3$) shows significant peaks as follows:

| δ | M | δ | M | δ | M |
|---|---|---|---|---|---|
| 181.2 | s | 134.8 | s | 65.0 | t |
| 178.1 | s | 132.1 | d | 62.3 | s |
| 172.1* | s | 129.7 | s | 56.8 | q |
| 171.5 | s | 124.4 | d | 56.5 | q |
| 165.8 | s | 120.8 | s | 42.9 | t |
| 162.1 | s | 119.9 | s | 41.9 | t |
| 155.5 | s | 119.5 | s | 25.8 | q |
| 153.4 | s | 117.7 | d | 25.5 | d |
| 150.8 | s | 107.3 | s | 22.4 | q |
| 148.7 | s | 104.9 | d | 22.3 | q |
| 140.7 | s | 100.4 | d | 20.2 | q |
| 137.7 | s | 93.4 | s | | |

*Two superimposed resonances.

## DETAILED DESCRIPTION OF THE INVENTION

The antibacterial agent LL-E19085α is produced by aerobic fermentation of microbial culture LL-E19085 which is a natural selection isolate of a culture isolated from a soil sample collected in Manyara, Tanzania. The culture was taxonomically characterized and identified as a new subspecies of *Micromonospora citrea.*

This new subspecies is maintained in the culture collection of the Medical Research Division, American Cyanamid Company, Pearl River, NY as culture number LL-E19085. A viable culture of this new microorganism has been deposited with the ARS Culture Collection, Fermentation Laboratory, Northern Regional Research Center, U. S. Department of Agriculture, 1815 North University Street, Peoria, IL 61604 and has been added to its permanent collection. It has been assigned the strain designation NRRL 18351 by such depository. Access to such culture, under strain designation NRRL 18351, during pendency of the instant application shall be available to one determined by the Commissioner of Patents and Trademarks to be entitled thereto under 37C.F.R. Section 1.14 and 35U.S.C. Section 122, and all restrictions on availability to

the public of such culture will be irrevocably removed upon grant of a patent on the instant application.

Observations were made of the cultural, physiological and morphological features of culture LL-E19085 using methods well known in the art. The generic assignment of LL-E19085 to the genus *Micromonospora* was confirmed morphologically and chemically. The strain produced monospores on the vegetative hyphae. No aerial hyphae were observed. Electron microscopic examination showed that the spores were warty. Whole cell analysis showed that LL-E19085 contained the *meso* isomer as well as traces of the L isomer of diaminopimelic acid. This strain showed the presence of xylose plus traces of arabinose in its whole cell sugar hydrolysates. Therefore LL-E19085 is considered to be a subspecies of *Micromonospora citrea.*

Comparative data on the morphology of LL-E19085 is given in Tables I and II. Physiological data is given in Tables III and IV.

TABLE I

| ISP Agar Medium | Spores | Vegetative Mycelium | Soluble Pigments |
|---|---|---|---|
| Yeast-Malt (ISP 2) | Slight, black at edge | Strong orange (50) to medium orange-yellow (71*) | Slight, brown-black |
| Oatmeal (ISP 3) | None | Light orange-yellow (70) to vivid orange-yellow (66) | Slight, brownish |
| Inorganic Salts-Starch (ISP 4) | None | Light orange-yellow (70) to vivid orange-yellow (66) | Slight, brownish |
| Glycerol-Asparagine (ISP 5) (54) | Slight, brownish at edge | Light tone of brownish-orange | Slight, brownish |

*Parenthetical numbers are colors taken from Kelly, K.L. and Judd, D. B., Color. Universal Language and Dictionary of Names, Nat. Bur. Stand. (U. S.), Spec. Publ. 440, 1976, Washington, D. C. and the accompanying Inter-Society Color Council, National Bureau of Standards Centroid Color Charts.

TABLE II

| Agar Medium | Actinomycete Growth (28°C, 2 weeks) |
|---|---|
| Pablum | Brown vegetative hyphae. Sparse spores. Soluble dark brown pigment. |
| Yeast Czapek's | Brownish tan vegetative hyphae. Sparse spores. Slight soluble dark-brownish pigment. |
| Czapek's | Vegetative hyphae covered/spores. Black spores. Slight dark pigment. |
| Yeast Extract-Dextrose | Black spores, Dry soluble brownish pigment. |
| Nutrient | Orange-brown vegetative hyphae. Sparse black spores. Moderate brown pigment. |
| Nutrient Glycerol | Blackish-tan vegetative hyphae. Sparse spores. Intense brown-black pigment. |
| Bennett's Dextrin | Tan vegetative hyphae. Moderate black spores. Soluble reddish-brown pigment. |
| Glucose Asparagine | Orange-tan vegetative hyphae. No spores. Slight soluble dark pigment. |

TABLE III

| Carbohydrate | Carbohydrate Utilization |
|---|---|
| Arabinose | + |
| Cellulose | - |
| Fructose | ± |
| Glucose | + |
| Inositol | - |
| Mannitol | - |
| Raffinose | - |
| Rhamnose | - |
| Sucrose | ± |
| Xylose | + |

TABLE IV

| Gordon Test | Physiological Reaction |
|---|---|
| **Hydrolysis of** | |
| Casein | + |
| Xanthine | - |
| Hypoxanthine | - |
| Tyrosine | + |
| Adenine | + |
| Gelatin | + |
| Potato Starch | + |
| Esculin | + |
| Physiological | |
| **Production of** | |
| Nitrate Reductase | - |
| Phosphatase | + |
| Urease | - |
| **Growth on** | |
| Salicin | - |
| 5% Sodium Chloride | - |
| Lysozyme Broth | - |
| **Decarboxylation of** | |
| Acetate | + |
| Benzoate | - |
| Citrate | - |
| Lactate | + |
| Malate | - |
| Mucate | - |
| Oxalate | - |
| Propionate | + |
| Pyruvate | + |
| Succinate | - |
| Tartrate | - |

TABLE IV (continued)

| Gordon Test | Physiological Reaction |
|---|---|
| **Acid from** | |
| Adonitol | - |
| Arabinose | + |
| Cellobiose | + |
| Dextrin | + |
| Dulcitol | - |
| Erythritol | - |
| Fructose | + |
| Galactose | + |
| Glucose | + |
| GLycerol | + |
| Inositol | - |
| Lactose | + |
| Maltose | + |
| Mannitol | - |
| Mannose | + |
| α-Methyl-D-glucoside | + |
| Melibiose | + |
| Raffinose | + |
| Rhamnose | - |
| Salicin | + |
| Sorbitol | - |
| Sucrose | + |
| Trehalose | + |
| Xylose | + |
| β-Methyl-D-xyloside | - |
| **Growth at** | |
| 10°C | - |
| 42°C | + |
| 45°C | + |

+ = positive; - = negative

It is to be understood that for the production of the new antibacterial agent LL-E19085α, the present invention is not limited to this particular organism or to organisms fully answering the above growth and microscopic characteristics, which are given for illustrative purposes only. In fact it is desired and intended to include the use of mutants produced from this organism by various means such as exposure to x-radiation, ultraviolet radiation, N′-methyl-N′-nitro-N-nitrosoguanidine, antinophages and the like.

The in vitro antibacterial effects of antibiotic LL-E19085α were determined by standard agar dilution methods against clinical isolates obtained from medical centers representing various geographical areas in the United States. The inoculum of each culture was approximately 1 to $5x10^4$ colony forming units applied with a Steers multiple inocula replicator to plates containing the antibiotic in Mueller-Hinton agar. The agar was supplemented with about 5% sheep blood where required for the growth of the organism. The results are given in Table V.

**TABLE V**

**In vitro Antibacterial Activity of LL-E19085α**

| Organism | | Minimal Inhibitory Concentration (mcg/ml) |
|---|---|---|
| *Staphylococcus aureus* | ATCC 25923 | 0.12 |
| *Staphylococcus aureus* | Smith | 0.12 |
| *Staphylococcus aureus* | VGH 84-45 | ≤0.06 |
| *Staphylococcus aureus* | CMC 83-127 | ≤0.06 |
| *Staphylococcus aureus* | CMC 83-131 | ≤0.06 |
| *Staphylococcus aureus* | CMC 83-132 | ≤0.06 |

TABLE V (continued)

| Organism | | Minimal Inhibitory Concentration (mcg/ml) |
|---|---|---|
| Staphylococcus aureus | SSC 82-57 | ≤0.06 |
| Staphylococcus epidermidis | IO 83-58 | ≤0.06 |
| Staphylococcus saphrophiticus | VGH 84-50 | ≤0.06 |
| Streptococcus β-hemolyticus | C 203 | ≤0.06 |
| Streptococcus β-hemolyticus | VGH 84-60 | ≤0.06 |
| Streptococcus β-hemolyticus | VGH 84-61 | ≤0.06 |
| Streptococcus β-hemolyticus | VGH 84-62 | ≤0.06 |
| Streptococcus pneumoniae | SV-1 | ≤0.06 |
| Streptococcus pneumoniae | K 84-21 | ≤0.06 |
| Enterococcus | VGH 84-65 | ≤0.06 |
| Enterococcus | VGH 84-68 | ≤0.06 |
| Enterococcus | IO 83-28 | ≤0.06 |
| Enterococcus | IO 83-40 | 0.12 |
| Enterococcus | CMC 83-72 | ≤0.06 |
| Escherichia coli | No. 311 | >128 |
| Klebsiella pneumoniae | AD | >128 |
| Enterobacter cloacae | VGH 84-37 | >128 |
| Morganella morganii | VGH 84-11 | >128 |
| Serratia marcescens | K 84-14 | >128 |
| Pseudomonas aeruginosa | 12-4-4 | >128 |
| Citrobacter diversis | MOR 84-3 | >128 |
| Proteus vulgaris | CMC 84-35 | >128 |
| Alcaligenes ssp. | ISG 86-34 | >128 |
| erratia rubidea | UHL 86-4 | >128 |
| Bacteroides fragilis | ATCC 25285 | 16 |
| Bacteroides vulgaris | ATCC 29327 | ≤0.06 |
| Bacteroides theta | ATCC 29741 | 4 |
| Bacteroides theta | ATCC 29742 | 4 |
| Clostridium perfringen | ATCC 13124 | ≤0.06 |
| Clostridium differensis | ATCC 17858 | ≤0.06 |
| Peptococcus magnus | ATCC 29328 | ≤0.06 |
| Peptococcus magnus | ATCC 14956 | ≤0.06 |
| Peptococcus asacrolytis | ATCC 29743 | ≤0.06 |
| Escherichia coli | ATCC 25922 | >128 |
| Staphylococcus aureus | ATCC 29213 | ≤0.06 |

## General Fermentation Conditions

Cultivation of Micromonospora citrea sp. LL-E19085 may be carried out in a wide variety of liquid culture media. Media which are useful for the production of antibiotic LL-E19085α include an assimilable source of carbon, such as starch, sugar, molasses, glycerol, etc.; an assimilable source of nitrogen, such as protein, protein hydrolysate, polypeptides, amino acids, cornsteep liquor, etc.; and inorganic anions and cations, such as potassium, sodium, ammonium, calcium, sulfate, carbonate, phosphate, chloride, etc. Trace elements such as boron, molybdenum, copper, etc., are supplied as impurities of other constituents of the medium. Aeration is supplied by forcing sterile air through or onto the surface of the fermenting medium.

Agitation is provided by a mechanical impeller. An antifoam agent may be added as needed. The growth of the organism is usually conducted at about 24-37°C, preferably at about 28°C.

The following examples describe the invention in detail.

## Example 1

### Inoculum Preparation

A typical medium used to grow the inocula was prepared according to the following formula:

| | |
|---|---|
| Dextrose..... | 1.0% |
| Dextrin..... | 2.0% |
| Yeast extract..... | 0.5% |
| NZ Amine A®*..... | 0.5% |
| Calcium carbonate..... | 0.1% |
| Defoam agent..... | 0.3% |
| Water.....qs..... | 100% |

*A pancreatic digest of casein, registered trademark of Sheffield Chemical, Norwich, NY.

This medium was sterilized and a 100ml portion in a 500ml flask was inoculated with mycelial scrapings from an agar slant of the culture *Micromonospora citrea* sp LL-E19085. The inoculated flask was then placed on a rotary shaker and agitated vigorously for approximately 48 hours at 32°C, providing primary inoculum.

A 100ml portion of this primary inoculum was then used to inoculate 10 liters of the above sterile medium which was incubated at 32°C with aeration for 72 hours, providing secondary inoculum.

A 10 liter portion of this secondary inoculum was then used to inoculate 260 liters of the above sterile medium in a tank. This medium was incubated at 32°C with agitation by an impeller driven at 180 rpm, a sterile air flow of 200 liters per minute and the addition of 50 ml of a defoaming agent for about 48 hours, providing tertiary inoculum.

## Example 2

### Fermentation

A fermentation medium was prepared according to the following formulation:

| | |
|---|---|
| Dextrin..... | 3.0% |
| Dextrose..... | 0.5% |
| Nutrisoy..... | 1.5% |
| Corn steep liquor..... | 0.5% |
| Calcium carbonate..... | 0.5% |
| Defoam agent..... | 0.3% |
| Water.....qs..... | 100% |

A 2800 liter portion of the above medium in a tank was sterilized and then inoculated with 300 liters of tertiary inoculum prepared as described in Example 1. Aeration was supplied at the rate of 6.5 liters of sterile air per liter of mash per minute and agitation was supplied by an impeller driven at about 110 rpm. The temperature was maintained at 28°C and defoaming agent was added as required. The fermentation was terminated after 129 hours.

## Example 3

### Isolation of Antibiotic LL-E19085α

A 1500 liter portion of the whole harvest mash, prepared as described in Example 2, was mixed with 15 liters of toluene for 30 minutes, then 250 lb of diatomaceous earth was added. After mixing for 15 minutes this mixture was filtered and the cake washed with 150 liters of water. The cake was slurried in a mixture of 208 liters of acetone, 416 liters of dichloromethane and 20 liters of 1.5N hydrochloric acid for 2 hours and then filtered. The cake was washed with about 175 liters of dichloromethane with the wash and filtrate combined. The cake was then washed with about 800 liters of water and this wash also combined with the above wash and filtrate and mixed. The dichloromethane layer was separated and washed with an equal volume of water. The dichloromethane layer was separated and concentrated to 100 liters, reextracted with fresh methylene chloride if any aqueous phase was present, and finally concentrated to about 1-3 liters.

The dichloromethane extracts were triturated repeatedly, first with hexane:dichloromethane (9:1) and then with hexane alone to remove the bulk of the fatty impurities giving a brown powder.

Several such partially purified preparations, from fermentations conducted as described in Example 2, totaling 20 g and averaging 10-30% LL-E19085α, were combined and purified by reverse-phase chromatography. The column consisted of a 15 liter bed of $C_{18}$ bonded phase packing of 40 micron particle size. The charge was loaded onto the column in 500 ml of acetonitrile:tetrahydrofuran (1:1). The column was developed at a flow rate of 1.0 liter per minute with a mobile phase consisting of acetonitrile:0.1M pH 4.5 ammonium acetate buffer (8:2). Fractions were collected at approximately 12 minute intervals. Fractions 6 and 7 were combined and evaporated, giving 2.7 g of pure LL-E19085α having the characteristics disclosed in the hereinabove specification.

## Claims

1. Antibiotic LL-E19085α, an antibacterial agent which has:
   a) molecular formula: $C_{36}H_{31}NO_{12}$;
   b) molecular weight: 669 FABMS;
   c) elemental analysis: C, 64.55; H, 4.46; N, 1.92;
   d) specific rotation $[\alpha]_D^{26}$ = -52° (C, 1.11% dichloromethane);
   e) ultraviolet absorption spectra: λmax nm ε 0.1N HCl = 224nm (27,700), 255nm (21,900), 328nm (16,400), 420nm (4,230)
   λmax nm ε 0.1N NaOH = 217nm (76,100), 340nm (15,100), 399nm (12,900)
   λmax nm ε $CH_3OH$ = 222nm (38,200), 240nm (30,100), 255nm (30,100), 321nm (24,500), 384nm (8,050)
   f) infrared absorption spectrum: (KBr disk), max ($cm^{-1}$) 1800, 1742, 1690, 1621, 1422, 1273;
   g) proton magnetic resonance spectrum: ($CDCl_3$) with significant peaks as follows:

| δ | #H | M | J(Hz) |
|---|---|---|---|
| 13.47 | 1 | s | - |
| 8.20 | 1 | d | 8.5 |
| 7.92 | 1 | d | 8.5 |
| 7.58 | 1 | s | - |
| 7.19 | 1 | s | - |
| 7.09 | 1 | s | - |
| 4.78 | 1 | d | 11.6 |
| 4.45 | 1 | d | 11.6 |
| 4.02 | 3 | s | - |
| 3.99 | 3 | s | - |
| 3.48 | 1 | d | 12.8 |
| 3.35 | 1 | d | 12.8 |
| 2.21 | 2 | d | 6.8 |
| 2.10 | 1 | m | - |
| 1.83 | 3 | s | - |
| 1.80 | 3 | s | - |
| 0.955 | 6 | d | 6.48; |

and

h) a carbon-13 nuclear magnetic resonance spectrum: ($CDCl_3$) with significant peaks as follows:

| δ | M | δ | M | δ | M |
|---|---|---|---|---|---|
| 181.2 | s | 134.8 | s | 65.0 | t |
| 178.1 | s | 132.1 | d | 62.3 | s |
| 172.1* | s | 129.7 | s | 56.8 | q |
| 171.5 | s | 124.4 | d | 56.5 | q |
| 165.8 | s | 120.8 | s | 42.9 | t |
| 162.1 | s | 119.9 | s | 41.9 | t |
| 155.5 | s | 119.5 | s | 25.8 | q |
| 153.4 | s | 117.7 | d | 25.5 | d |
| 150.8 | s | 107.3 | s | 22.4 | q |
| 148.7 | s | 104.9 | d | 22.3 | q |
| 140.7 | s | 100.4 | d | 20.2 | q |
| 137.7 | s | 93.4 | s | | |

*Two superimposed resonances.

2. A method of treating bacterial infections in warm-blooded animals which comprises administering to said animals an antibacterially effective amount of LL-E19085α.

3. A process for producing antibiotic LL-E19085α which comprises aerobically fermenting the microorganism *Micromonospora citrea* sp. NRRL 18351 or mutants thereof in a liquid medium containing assimilaable sources of carbon, nitrogen and inorganic salts, until substantial antibiotic activity is imparted to said medium and then recovering the antibiotic therefrom.

4. The process for producing antibiotic LLE19085α as recited in Claim 3, which comprises aerobically fermenting a liquid medium containing assimilable sources of carbon, nitrogen and inorganic salts, which medium has been inoculated with a viable culture of the microorganism *Micromonospora citrea* sp. NRRL 18351 or mutants thereof, maintaining said fermentation culture at a temperature of about 24-37°C for a period of 50-150 hours, harvesting the mash and extracting the antibiotic.

5. A biologically pure culture of the microorganism *Micromonospora citrea* sp. NRRL 18351.

6. A biologically pure culture of the microorganism *Micromonospora citrea* sp. wherein said microorganism has spontaneously mutated, such that the microorganism is genetically altered but still retains the ability to synthesize antibiotic LL-E19085α.

7. A biologically pure culture of the microorganism *Micromonospora citrea* sp. wherein said microorganism has been subjected to mutagenic means such that the microorganism is genetically altered but still retains the ability to synthesize antibiotic LL-E19085α.